(19) Europäisches Patentamt
European Patent Office
Office européen des brevets

(11) Publication number: **0 064 820**
**A1**

(12) # EUROPEAN PATENT APPLICATION

(21) Application number: **82301971.6**

(22) Date of filing: **16.04.82**

(51) Int. Cl.³: **C 07 D 233/56**
C 07 D 233/58, C 07 D 233/06
C 07 D 233/08, A 61 K 31/415

(30) Priority: **22.04.81 GB 8112506**

(43) Date of publication of application:
**17.11.82 Bulletin 82/46**

(84) Designated Contracting States:
**AT BE CH DE FR GB IT LI LU NL SE**

(71) Applicant: **Farmos-Yhtymä Oy**
**P.O. Box 425**
**SF-20101 Turku 10(FI)**

(72) Inventor: **Karjalainen, Arto Johannes**
**Myllyojantie 13 H 37**
**SF-90650 Oulu 65(FI)**

(72) Inventor: **Kurkela, Kauko Oiva Antero**
**Keulatie 4**
**SF-90560 Oulu 56(FI)**

(74) Representative: **Collier, Jeremy Austin Grey et al,**
**J.A.Kemp & Co. 14, South Square Gray's Inn**
**London WC1R 5EU(GB)**

(54) **Substituted imidazole and imidazoline derivatives and their preparation and use.**

(57) The invention provides novel compounds of the formula:

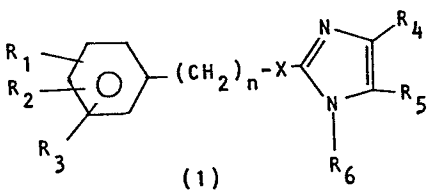

(1) or

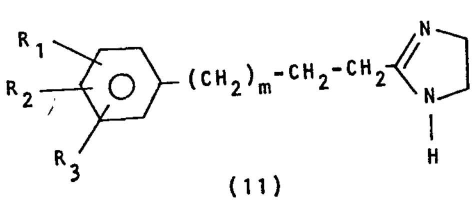

(11)

wherein each of $R_1$, $R_2$ and $R_3$, which can be the same or different, is hydrogen, chloro, bromo, fluoro, methyl, ethyl, methoxy, amino, hydroxy or nitro; $R_4$ and $R_5$, which can be the same or different, are hydrogen, an alkyl radical of 1 to 7 carbon atoms, hydroxymethyl or

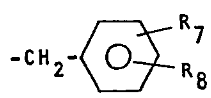

;

$R_6$ is hydrogen, an alkyl radical of 1 to 7 carbon atoms or

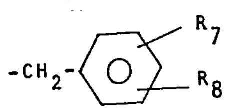

;

$R_7$ and $R_8$ are hydrogen, chloro or methyl; X is $-CH=CH-$ or $CHR_9$; $R_9$ is hydrogen or hydroxy; n is 0-7 and m is 0-6; and their non-toxic pharmaceutically acceptable acid addition salts and mixtures thereof. Processes for the preparation of these compounds are described, as are novel pharmaceutical compositions comprising at least one of the compounds and their salts. The compounds and their non-toxic salts exhibit valuable pharmacological activity and are useful in the treatment of mammals, especially as antihypertensives and also as antithrombotic and antimycotic agents.

EP 0 064 820 A1

## DESCRIPTION

## "SUBSTITUTED IMIDAZOLE AND IMIDAZOLINE DERIVATIVES AND THEIR PREPARATION AND USE"

The present invention relates to 2-substituted imidazole and imidazoline derivatives and their non-toxic, pharmaceutically acceptable acid addition salts, and their preparation, to pharmaceutical compositions containing the same, and to their use.

The imidazole and imidazoline derivatives of the present invention have the general formula:

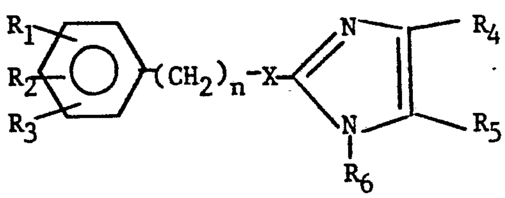

or

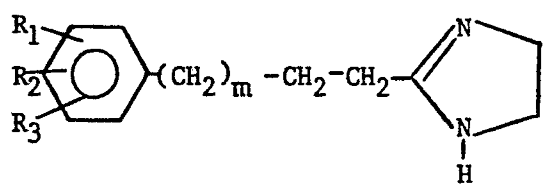

(I) (II)

wherein each of $R_1$, $R_2$ and $R_3$, which can be the same or different, is hydrogen, chloro, bromo, fluoro, methyl, ethyl, methoxy, amino, hydroxy or nitro; $R_4$ and $R_5$, which can be the same or different, are hydrogen, an alkyl radical of 1 to 7 carbon atoms, hydroxymethyl or

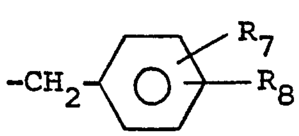;

$R_6$ is hydrogen, an alkyl group of 1-7 carbon atoms

or 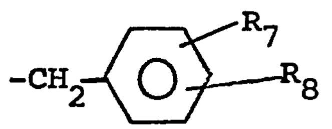 ;

$R_7$ and $R_8$ are hydrogen, chloro or methyl;

X is -CH=CH- or $-CHR_9-$; $R_9$ is hydrogen or hydroxy; n is 0-7 and m is 0-6. Because of the tautomerism in the imidazole ring the compounds of the general formula I (when $R_6$=H) are 4(5)-substituted imidazole derivatives.

The compounds of formulae I and II are novel provided that, in general formula I

a) when n = 0 and $R_1$ and $R_2$ are both hydrogen then $R_3$ is not hydrogen, methoxy or 4-chloro,

b) when n = 0 and X = -CHOH -, then $R_6$ is not methyl,

c) when n = 0, X = -CH=CH-, and $R_1$ and $R_2$ are hydrogen, $R_3$ is not nitro, amino, or hydroxy

d) when n is 1 and $R_1$, $R_2$, $R_3$ and $R_5$ are all hydrogen, then if X is $CH_2$ and $R_6$ is hydrogen, $R_4$ is other than hydroxymethyl or if X is CHOH and $R_4$ is hydrogen, $R_6$ is other than methyl, and

e) when n is 2 and X is $CH_2$ then $R_1$, $R_2$, $R_3$, $R_4$, $R_5$ and $R_6$ are not all hydrogen, and

f) in general formula (II),

when m is 0 or 1, $R_1$, $R_2$ and $R_3$ are not all hydrogen.

The non-toxic pharmaceutically acceptable acid addition salts of these compounds are also within the scope of the invention. The compounds of the formula (I) and (II) form acid addition salts with both organic and inorganic acids. They can thus form many pharmaceutically usable acid addition salts, as, for instance, chlorides, bromides, sulfates, nitrates, phosphates, sulfonates, formates, tartrates, maleates, citrates, benzoates, salicylates, ascorbates and the like.

The invention includes within its scope pharmaceutical compositions comprising at least one of the compounds of formula (I) and (II) or a non-toxic pharmaceutically acceptable salt thereof, and a compatible pharmaceutically acceptable carrier therefor.

The present invention provides, for example, the following specific compounds of formula (I), wherein X represents $-CH_2-$ :

2-(2,6-dimethylbenzyl)-imidazole
2-(2,3-dimethylbenzyl)-imidazole
2-(2,4-dimethylbenzyl)-imidazole
2-(3,4-dimethylbenzyl)-imidazole
2-(2,5-dimethylbenzyl)-imidazole
2-(2,6-dichlorobenzyl)-imidazole
2-(2,3-dichlorobenzyl)-imidazole
2-(2,4-dichlorobenzyl)-imidazole
2-(3,4-dichlorobenzyl)-imidazole
2-(2,5-dichlorobenzyl)-imidazole
2-(2,6-dibromobenzyl)-imidazole
2-(2,6-difluorobenzyl)-imidazole
2-(2-methylbenzyl)-imidazole
2-(4-methylbenzyl)-imidazole
2-(4-ethylbenzyl)-imidazole
2-(2-chlorobenzyl)-imidazole
2-(4-chlorobenzyl)-imidazole
2-(2,6-dimethylbenzyl)-4-methyl-imidazole
2-(2,6-dimethylbenzyl)-4,5-dimethyl-imidazole
2-(2,6-dimethylbenzyl)-4-hydroxymethyl-imidazole
2-(2-methylbenzyl)-4-methyl-imidazole

2-(2-methylbenzyl)-4,5-dimethyl-imidazole

2-(2-methylbenzyl)-4-hydroxymethyl-imidazole

2-(2,6-dimethylbenzyl)-4-ethyl-imidazole

2-(3,4-dimethoxybenzyl)-4-methyl-imidazole

2-(3,4-dihydroxybenzyl)-4-methyl-imidazole

2-(3-methoxybenzyl)-4-methyl-imidazole

2-(3-hydroxybenzyl)-4-methyl-imidazole

2-[2-(2,6-dimethylphenyl)ethyl]-imidazole

2-[2-(2,3-dimethylphenyl)ethyl]-imidazole

2-[2-(2,4-dimethylphenyl)ethyl]-imidazole

2-[2-(3,4-dimethylphenyl)ethyl]-imidazole

2-[2-(2,5-dimethylphenyl)ethyl]-imidazole

2-[2-(2,6-dichlorophenyl)ethyl]-imidazole

2-[2-(2,3-dichlorophenyl)ethyl]-imidazole

2-[2-(2,4-dichlorophenyl)ethyl]-imidazole

2-[2-(3,4-dichlorophenyl)ethyl]-imidazole

2-[2-(2,5-dichlorophenyl)ethyl]-imidazole

2-[2-(2,6-dibromophenyl)ethyl]-imidazole

2-[2-(2,6-difluorophenyl)ethyl]-imidazole

2-[2-(3,4-dihydroxyphenyl)ethyl]-imidazole

2-[2-(3,4-dimethoxyphenyl)ethyl]-imidazole

2-[2-(2-methylphenyl)ethyl]-imidazole

2-[2-(3-methylphenyl)ethyl]-imidazole

2-[2-(4-methylphenyl)ethyl]-imidazole

2-[2-(4-ethylphenyl)ethyl]-imidazole

2-[2-(2-chlorophenyl)ethyl]-imidazole

2-[2-(3-chlorophenyl)ethyl]-imidazole

2-[2-(2,6-dimethylphenyl)ethyl]-4-methyl-imidazole

2-[2-(2,3-dimethylphenyl)ethyl]-4-methyl-imidazole

2-[2-(2,6-dimethylphenyl)ethyl]-4-ethyl-imidazole

2-[2-(2,3-dimethylphenyl)ethyl]-4-ethyl-imidazole

2-[2-(2-methylphenyl)ethyl]-4-methyl-imidazole

2-[2-(2-chlorophenyl)ethyl]-4-methyl-imidazole

2-[2-(4-chlorophenyl)ethyl]-4-methyl-imidazole

2-[2-(2,6-dichlorophenyl)ethyl]-4-methyl-imidazole

2-[2-(2,6-dimethylphenyl)ethyl]-4,5-dimethyl-imidazole

2-[2-(2,6-dimethylphenyl)ethyl]-4,5-diethyl-imidazole

2-[2-(2,6-dimethylphenyl)ethyl]-4-ethyl-5-methyl-imidazole

2-[2-(2,6-dimethylphenyl)ethyl]-4-(2,6-dimethylbenzyl)-imidazole

2-[2-(3,4-dimethoxyphenyl)ethyl]-4-methyl-imidazole

2-[2-(3,4-dihydroxyphenyl)ethyl]-4-methyl-imidazole

2-[3-(2,6-dimethylphenyl)propyl]-imidazole

2-[3-(2,3-dimethylphenyl)propyl]-imidazole

2-[3-(2,4-dimethylphenyl)propyl]-imidazole

2-[3-(2,5-dimethylphenyl)propyl]-imidazole

2-[3-(2,6-dichlorophenyl)propyl]-imidazole

2-[3-(2,4-dichlorophenyl)propyl]-imidazole

2-[3-(2,6-dimethylphenyl)propyl]-4-methyl-imidazole

2-[3-(2,3-dimethylphenyl)propyl]-4-methyl-imidazole

2-[3-(2,4-dimethylphenyl)propyl]-4-methyl-imidazole

2-[3-(2,3-dimethylphenyl)propyl]-4-ethyl-imidazole

2-[3-(2,6-dichlorophenyl)propyl]-imidazole

2-[3-(2,4-dichlorophenyl)propyl]-imidazole

2-[3-(2,6-dichlorophenyl)propyl]-4-methyl-imidazole

2-[3-(2,4-dichlorophenyl)propyl]-4-methyl-imidazole

2-[4-(2,6-dimethylphenyl)butyl]-imidazole

2-[4-(2,3-dimethylphenyl)butyl]-imidazole

2-[4-(2,6-dichlorophenyl)butyl]-imidazole

2-[5-(2,6-dimethylphenyl)pentyl]-imidazole

2-[5-(3,4-dimethylphenyl)pentyl]-imidazole

1-Ethyl-2-[2-(2,6-dimethylphenyl)ethyl]-imidazole

1-Methyl-2-[3-(2,6-dimethylphenyl)propyl]-imidazole

The present invention also provides for example the following specific compounds of formula (I) wherein X represents -CHOH- :

2-[α-(2,6-dimethylphenyl)hydroxymethyl]-imidazole

2-[α-(2,3-dimethylphenyl)hydroxymethyl]-imidazole

2-[2-(2,6-dimethylphenyl)-1-hydroxyethyl]-imidazole

2-[2-(2,3-dimethylphenyl)-1-hydroxyethyl]-imidazole

2-[2-(3,4-dimethylphenyl)-1-hydroxyethyl]-imidazole

2-[2-(2,5-dimethylphenyl)-1-hydroxyethyl]-imidazole

2-[3-(2,6-dimethylphenyl)-1-hydroxypropyl]-4-methyl-imidazole

2-[3-(2,6-dimethylphenyl)-1-hydroxypropyl]-4,5-dimethyl-imidazole

2-[3-(2,6-dimethylphenyl)-1-hydroxypropyl]-imidazole

1-Benzyl-2-[2-(3,4-dimethylphenyl)-1-hydroxyethyl]-imidazole

1-Benzyl-2-[3-(2,6-dimethylphenyl)-1-hydroxypropyl]-imidazole

1-Benzyl-2-[α-(2,6-dimethylphenyl)-hydroxymethyl]-imidazole

1-Benzyl-2-[α-(2,3-dimethylphenyl)-hydroxymethyl]-imidazole

2-[5-(2,6-dimethylphenyl)-1-hydroxypentyl]-imidazole

1-Methyl-2-[3-(2,6-dimethylphenyl)-1-hydroxypropyl]-imidazole

1-Ethyl-2-[2-(2,3-dimethylphenyl)-1-hydroxyethyl]-imidazole

The present invention provides e.g. the following compounds of formula (I) wherein X is -CH=CH-:

2-[2-(3,4-dimethylphenyl)-ethenyl]-imidazole

2-[2-(2,5-dimethylphenyl)-ethenyl]-imidazole

2-[2-(2,6-dimethylphenyl)-ethenyl]-imidazole

2-[2-(2,3-dimethylphenyl)-ethenyl]-imidazole

2-[2-(2,6-dichlorophenyl)-ethenyl]-imidazole

2-[2-(2,6-dimethylphenyl)-ethenyl]-1-methyl-imidazole

1-Benzyl-[2-(2,6-dimethylphenyl)ethenyl]-imidazole

2-[2-(2,6-dimethylphenyl)-ethenyl]-4-methyl-imidazole

2-[2-(2,6-dimethylphenyl)-ethenyl]-4,5-dimethyl-imidazole

2-[2-(2,6-dimethylphenyl)-ethenyl]-4-ethyl-imidazole

2-[2-(2,6-dimethylphenyl)-ethenyl]-4-methyl-5-ethyl-imidazole

2-[3-(2,6-dimethylphenyl)-1-propenyl]-imidazole

2-[3-(2,3-dimethylphenyl)-1-propenyl]-imidazole

2-[3-(2,6-dichlorophenyl)-1-propenyl]-imidazole

2-[4-(2,6-dimethylphenyl)-1-butenyl]-imidazole

2-[4-(2,3-dimethylphenyl)-1-butenyl]-imidazole

2-[4-(2,6-dichlorophenyl)-1-butenyl]-imidazole

2-[5-(2,6-dimethylphenyl)-1-pentenyl]-imidazole

2-[5-(2,6-dichlorophenyl)-1-pentenyl]-imidazole

2-[6-(2,6-dimethylphenyl)-1-hexenyl]-imidazole

2-[6-(2,6-dichlorophenyl)-1-hexenyl]-imidazole

The present invention provides for example the following imidazoline derivatives:

2-[2-(2,6-dimethylphenyl)-ethyl]-imidazoline

2-[2-(2,5-dimethylphenyl)-ethyl]-imidazoline

2-[2-(2,3-dimethylphenyl)-ethyl]-imidazoline

2-[3-(2,6-dimethylphenyl)-propyl]-imidazoline

2-[3-(3-methylphenyl)-propyl]-imidazoline

2-[4-(2,6-dimethylphenyl)-butyl]-imidazoline

2-[4-(2,6-dichlorophenyl)-butyl]-imidazoline

2-[5-(2,6-dimethylphenyl)-pentyl]-imidazoline

2-[5-(2,3-dimethylphenyl)-pentyl]-imidazoline

The compounds of the present invention have been found to possess excellent antihypertensive

properties and are also effective antithrombotic agents. The compounds of the formula (I) and (II) can be prepared according to several processes, namely

A. (The Grignard reaction)

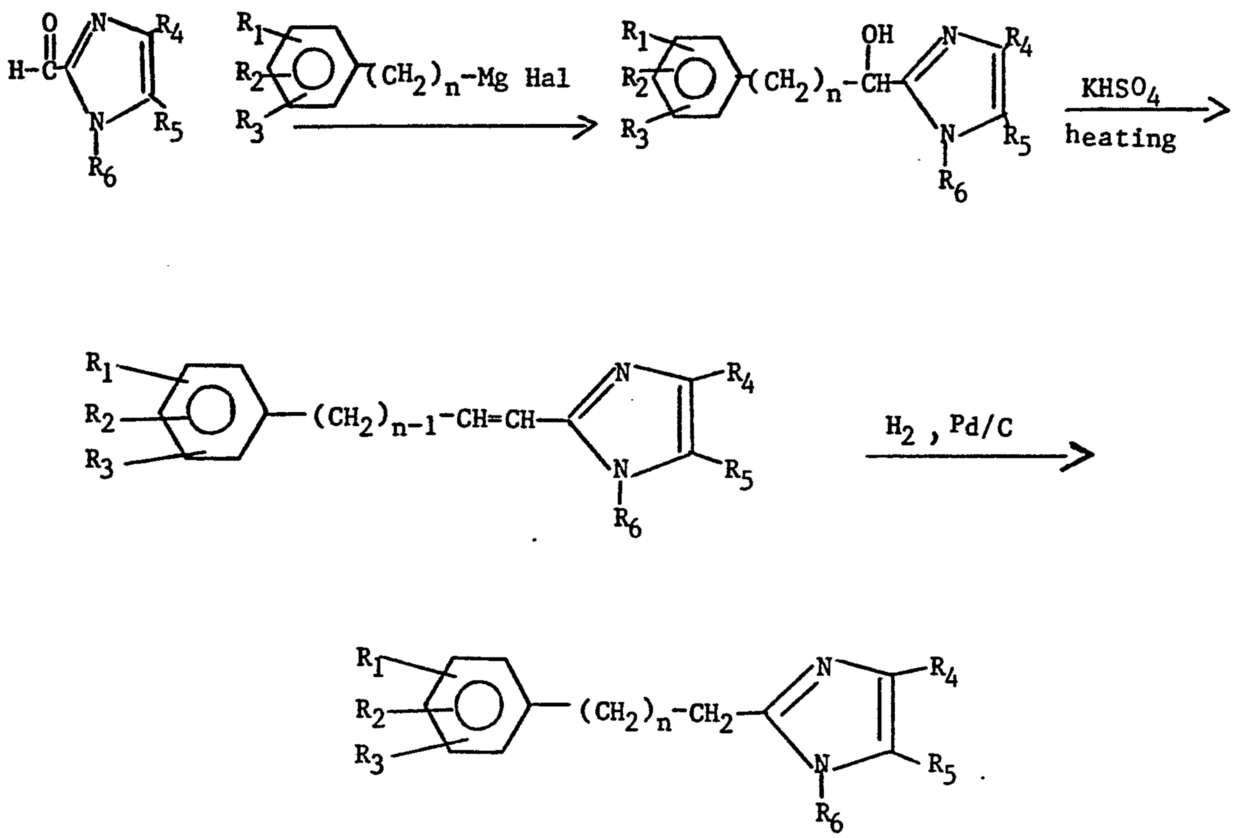


$R_6$ can also be in the 3-position of the imidazole ring. When $R_6$ is a benzyl group it can be removed with sodium in liquid ammonia:

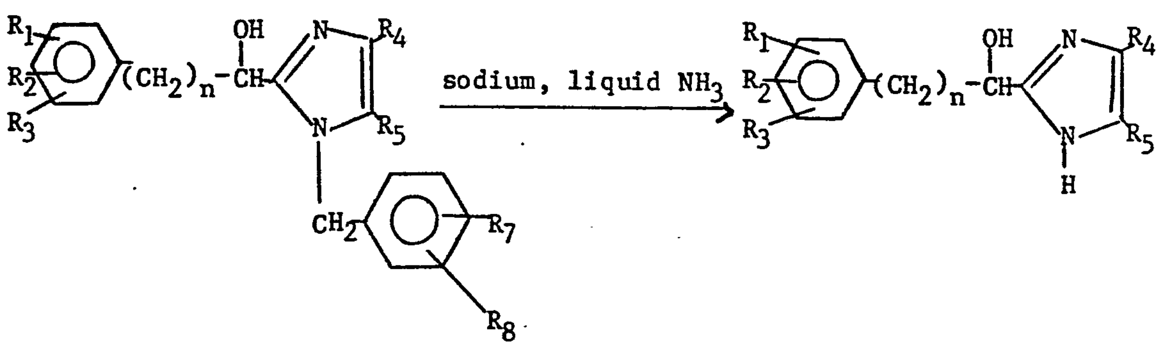

after which follows a dehydration process with $KHSO_4$ as above, and the formed double bond is hydrogenated to a compound of the formula

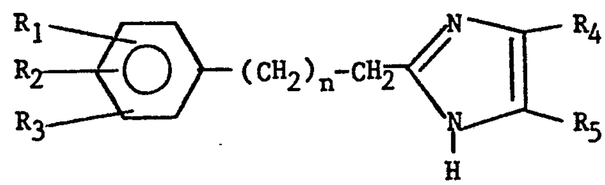

B. (The Wittig reaction)

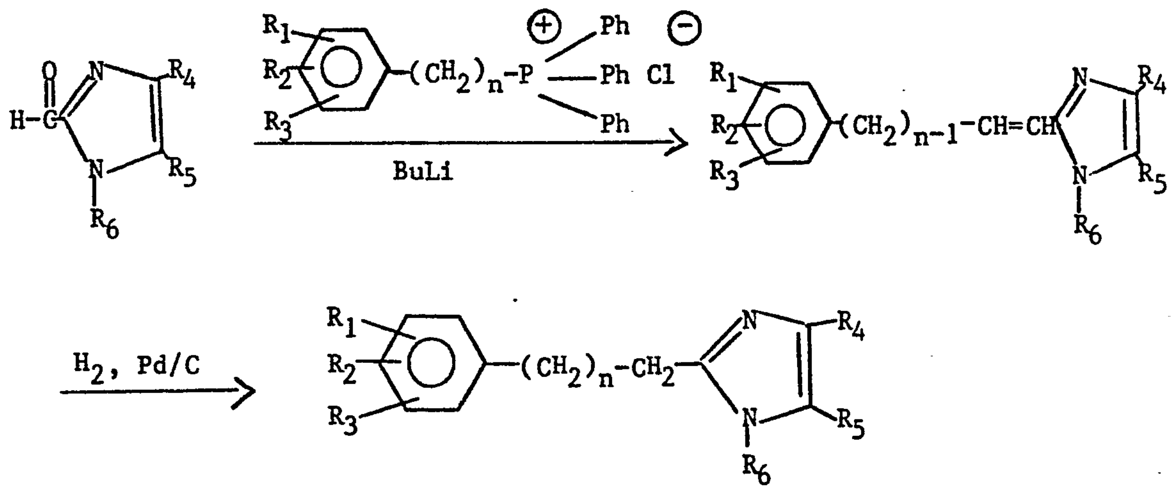


$R_6$ can also be in the N3-position.

When $R_6$ is a benzyl group it can be removed with sodium in liquid ammonia yielding the compound:

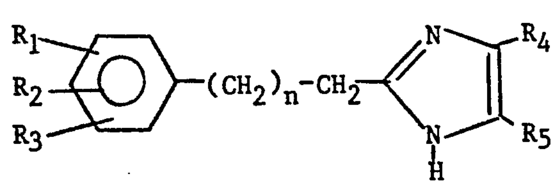

C.

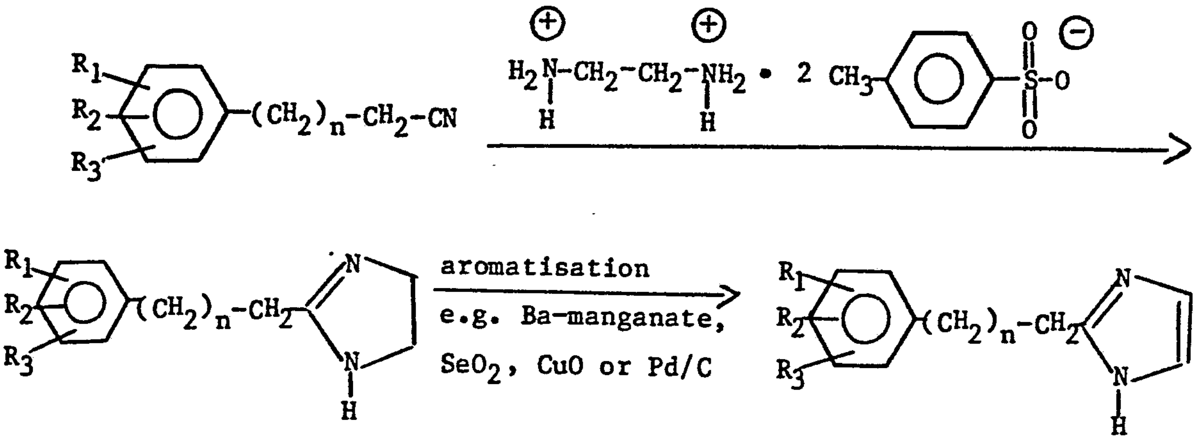
aromatisation
e.g. Ba-manganate,
$SeO_2$, CuO or Pd/C

D.

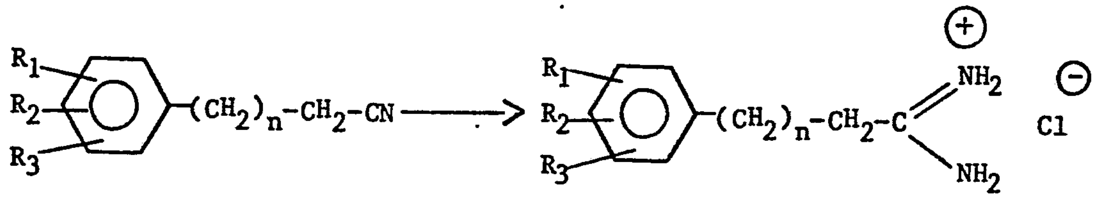

e.g.

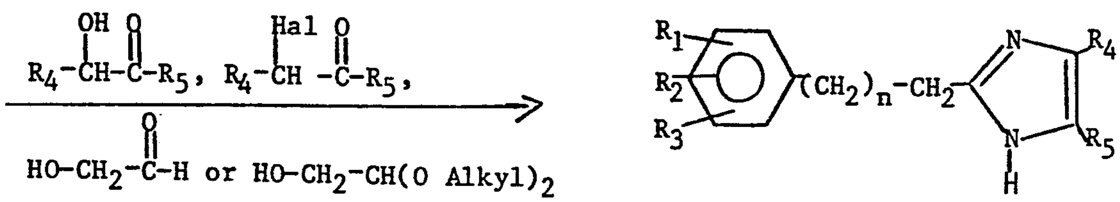
or

E.

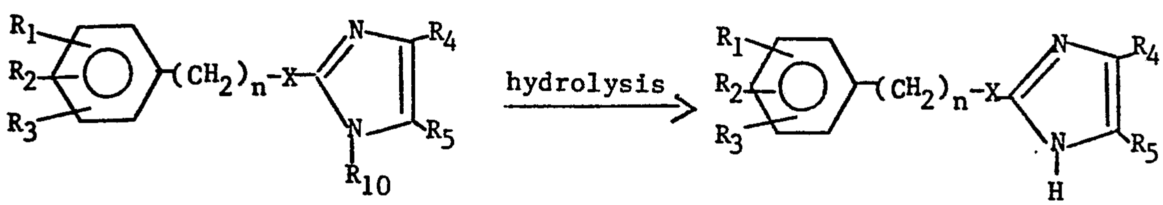
hydrolysis

wherein $R_{10}$ is a hydrolysable group as for instance:
-CO Alkyl, wherein the alkyl group contains 1 to 7 carbon atoms,

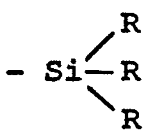 , wherein R is a lower alkyl group or phenyl

or

- $CH(O\ Alkyl)_2$, wherein the alkyl group contains 1 to 7 carbon atoms

The N-substituent $R_{10}$ can also be in the 3-position.

F.

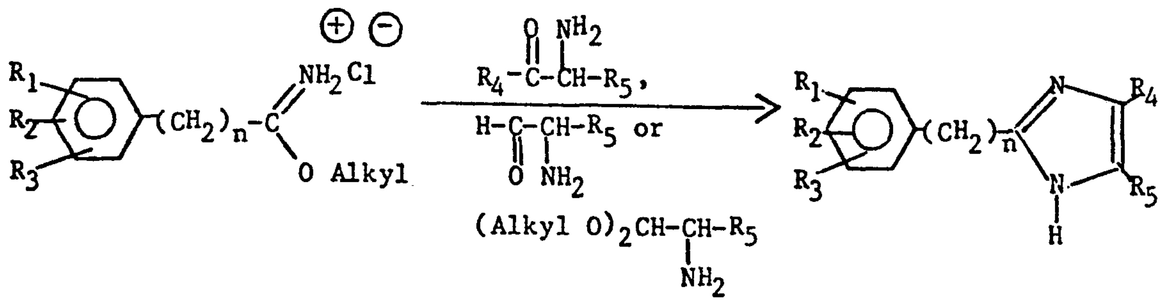


Process A (Grignard reaction)

In the first step the solvent is preferably diethylether or tetrahydrofuran and the temperature varies from about 40°C to the boiling point of tetrahydrofuran.
The second step is a dehydration step and can be performed e.g. by heating with potassiumhydrogensulfate to about 100-200°C.
In the third step the double bond is hydrogenated by conventional methods, e.g. using Pd on carbon as

catalyst and e.g. aqueous ethanol, ethanol or tetrahydrofuran as solvent.

If $R_6$ is a benzyl group it can be removed at a low temperature, e.g. 0° to -40°C, with metallic sodium in liquid ammonia.

Process B is a Wittig reaction comprising the reaction of an N-substituted imidazole-2-aldehyde with an aralkylidenetriphenyl-phosphorane, which is prepared from the corresponding aralkyltriphenyl-phosphonium halide and a basic agent, preferably butyllithium. The reaction is performed in an inert solvent, e.g. tetrahydrofuran, at elevated temperature. In the steps 2 and 3 of process B the formed double bond is hydrogenated with conventional methods and the benzyl group can be removed as in process A.

In process C an arylalkylimidazoline derivative is aromatised to the corresponding imidazole derivative.

In the first stage the imidazoline compound is synthesized by heating the corresponding arylalkylnitrile and a salt of ethylenediamine, preferably the p-toluenesulfonate salt, to 150-200°C. In the second step the imidazoline derivative is reacted in a suitable solvent, e.g. methylene chloride, at elevated temperature with e.g. barium manganate, $SeO_2$, CuO or palladium on carbon.

According to process D an arylalkylnitrile is converted to the corresponding imidamide by reaction first with

HCl-gas under dry conditions in absolute ethanol at a low temperature, about 0°C, and then with ethanolic ammonia preferably at room temperature. In the second step the imidamide derivative is reacted with an acetaldehyde derivative at room temperature in a suitable solvent, e.g. a lower alcohol or an aqueous alcohol. Suitable acetaldehyde derivatives are hydroxyacetaldehyde, halogenacetaldehyde and their dimethyl- and diethylacetals.

The hydrolysis according to process E is performed according to conventional methods.

Process F concerns a process in which imino ethers are reacted with aminoketones. Aminoacetaldehydes and their acetal derivatives can also be used. Especially useful is e.g. the dimethylacetal of aminoacetaldehyde of the formula $(MeO)_2CHCH_2NH_2$. The reaction is performed e.g. by refluxing in isopropanol.

As stated herein above, the compounds of the general formula (I) and (II) and their non-toxic, pharmaceutically acceptable acid addition salts have valuable pharmacological properties and have been found to possess excellent anti-hypertensive activity in mammals. This activity makes these imidazole derivatives particularly useful in the treatment of high blood pressure.

Administration of isomeric compounds of

formula (I), their non-toxic, pharmaceutically acceptable acid salts or mixtures thereof may be achieved parenterally, intravenously or orally. Typically, an effective amount of the derivative is combined with a suitable pharmaceutical carrier. As used herein, the term "effective amount" encompasses those amounts which yield the desired activity without causing adverse side-effects. The precise amount employed in a particular situation is dependent upon numerous factors such as method of administration, type of mammal, condition for which the derivative is administered, etc., and of course the structure of the derivative. When used for their anti-hypertensive activity the compounds of the invention are generally administered orally in a daily dosage of 0.5 to 5 mg/kg of treated mammal.

The pharmaceutical carriers which are typically employed with the derivatives of the present invention may be solid or liquid and are generally selected with the planned manner of administration in mind. Thus, for example, solid carriers include lactose, sucrose, gelatin and agar, while liquid carriers include water, syrup, peanut oil and olive oil. Other suitable carriers are well-known to those skilled in the art of pharmaceutical formulations. The combination of the derivative and the carrier may be fashioned into numerous acceptable forms, such as tablets, capsules, suppositories, solutions, emulsions and powders.

The anti-hypertensive properties of the

imidazole derivatives of the present invention have been determined according to the following procedure. Sprague-Dawley rats of normal weight were first anesthetized with urethane. After this, the femoral artery was connected by way of a polyethylene tube with a blood pressure transducer. The test substance was then injected into the femoral vein and the blood pressure and the pulse frequency were registered with a recorder.

The antithrombotic activity was investigated in vitro. The inhibiting activity of the compounds against ADP- and collagen-induced aggregation of thrombocytes was measured. In the test thrombocytes from a cow were used. To 1.2 ml of plasma containing 250000 thrombocytes/$mm^3$ were added 50 µl of a solution of the compound to be tested. After 10 min incubation either ADP or collagen was added. The aggregation of the thrombocytes was turbidimetrically determined at $\lambda$ = 605 nm.

Acute toxicity was determined by using female mice of NMRI-Strain with an age of about 7 months and weighing 30-40 g. The administration of the test compound was i.v.

In the pharmacological examination of the compounds of the invention for example the following results have been obtained.

In the above described anti-hypertension test the compound 2-[2-(2,6-dimethylphenyl)-ethyl]-imidazole lowered the blood pressure for a shorter time with a dose of 30 and 100 µg/kg i.v. in rats. The dose 300 µg/kg i.v. gave a lowered blood pressure of long duration. With the dose 3 mg/kg i.v. the decrease of the blood pressure was 30%. Doses of 30 mg/kg i.v. did not elevate the blood pressure. At high dosages a slight decrease of the pulse frequency can be observed. When the compound is administered by i.c.v. dosage the blood pressure is lowered with 10 x smaller amounts. The $LD_{50}$ i.v. in mice of the compound is 20 mg/kg.

The compound 2-[2-(2,6-dimethylphenyl)ethyl]-4-methyl-imidazole lowered the blood pressure with doses of 10 µg/kg - 10 mg/kg. A dosage of 300 µg/kg i.v. lowered the blood pressure by 45%. The $LD_{50}$ is 25 mg/kg i.v. in mice.

The compound 2-[2-(2,6-dimethylphenyl)-1-hydroxyethyl]-imidazole causes a lowering of the blood pressure of short duration at a dose of 1 and 3 mg/kg i.v. The dose 10 mg/kg lowers the blood pressure for a longer period. The pulse frequency is very slightly influenced. The $LD_{50}$ is 60 mg/kg i.v. in mice.

The compound 2-[2-(2,6-dimethylphenyl)-ethenyl]-imidazole lowers the blood pressure with a dose of 3 mg/kg i.v. in rats by 17 per cent. The compound has no effect

on the pulse frequency. The $LD_{50}$ in mice i.v. is 40 mg/kg. The compound 2-[2-(2,6-dimethylphenyl)-ethyl]-imidazoline, which has a $LD_{50}$ of 25 mg/kg i.v. in mice, lowers the blood pressure with a dose of 1-3 mg/kg by ·20 % in rats.
The compound 2-[2-(2,5-dimethylphenyl)-ethyl]-imidazole ($LD_{50}$ in mice i.v. = 30 mg/kg) lowered the blood pressure with dosages beginning from 3 mg/kg. The maximal decrease of the blood pressure was 37 % and this was obtained with a dose of 10 mg/kg i.v.
The compound 2-[2-(3,4-dimethylphenyl)ethyl]-imidazole ($LD_{50}$ in mice i.v. = 40 mg/kg) lowered the blood pressure by 20 % with a dose of 10 mg/kg.
The compound 2-[2-(3,4-dimethylphenyl)-1-hydroxyethyl]-imidazole ($LD_{50}$ in mice i.v. = 50 mg/kg) lowered the blood pressure with doses beginning from 1 mg/kg, the maximal decrease being 17 %.
The compound 2-[2-(2,3-dimethylphenyl)ethyl]-4-methyl-imidazole ($LD_{50}$ in mice i.v. = 35 mg/kg) lowered the blood pressure with doses from 0.1 to 10 mg/kg, the maximal decrease being 32 %.
The compound 2-[2-(2,3-dimethylphenyl)ethyl]-imidazoline ($LD_{50}$ in mice i.v. = 30 mg/kg) lowered the blood pressure with doses of 3 to 10 mg/kg, the maximal decrease being 26%.

In the antithrombotic activity test, the following compounds inhibited the collagen-induced and the ADP-induced aggregation of thrombocytes effectively:

2-[2-(2,6-dimethylphenyl)-ethyl]-imidazoline

2-[2-(2,3-dimethylphenyl)-ethyl]-imidazoline

2-[2-(2,6-dimethylphenyl)-ethyl]-imidazole

2-[2-(2,3-dimethylphenyl)-ethyl]-4-methyl-imidazole.

1-Benzyl-2-[3-(2,6-dimethylphenyl)-1-hydroxypropyl]-imidazole. ($LD_{50}$ in mice i.v. = 65 mg/kg)

2-[2-(2,5-dimethylphenyl)-1-hydroxyethyl]-imidazole. ($LD_{50}$ in mice i.v. = 45 mg/kg)

2-(2-methylbenzyl)-4,5-dimethyl-imidazole. ($LD_{50}$ in mice i.v. = 30 mg/kg).

In the Examples below, where $1_H$-NMR spectrum shifts are presented, the NMR spectra were determined with a Perkin-Elmer R 24 apparatus using an external tetramethylsilane standard, from which the presented chemical shifts (δ, ppm) are tabulated. The letters s, d, t and m are used to indicate a singlet, doublet, triplet or multiplet, respectively. In the same connection, the number of hydrogen atoms is also stated. The compounds which are indicated as bases are tested in deuterium methanol, deuterium acetone or deuterium chloroform, while the values for compounds which are indicated as hydrochlorides were determined in deuterium

oxide. The presented $^{13}$C-NMR-spectrum were determined with a Bruker WB-80 DS apparatus.

Example 1

2-[2-(2,6-dimethylphenyl)-ethyl]-imidazole

a) 1-Benzyl-2-[2-(2,6-dimethylphenyl)-1-hydroxyethyl]-imidazole

The Grignard reagent of 9.6 g 2,6-dimethyl-benzyl-chloride is prepared by adding 1.5 g Mg turnings to an ethereal solution thereof. 9.2 g N-benzyl-2-imidazole-aldehyde is dissolved in 50 ml tetrahydrofuran, the solution is heated to about 40°C and the above mentioned Grignard-solution is added dropwise. The mixture is refluxed for 3 hours and poured in ice-water containing hydrogen chloride and the solution is washed with toluene. The solution is made alkaline and the product extracted with ethyl acetate. After distilling off the solvent a residue containing crude product is obtained, which can be recrystallized from acetone-water.

b) 2-[2-(2,6-dimethylphenyl)-1-hydroxyethyl]-imidazole

3.5 g of 1-benzyl-2-[2-(2,6-dimethylphenyl)-1-hydroxyethyl]-imidazole obtained in step a) is suspended in a mixture of 10 ml liquid ammonia and 10 ml ether. To the mixture is added in small portions by stirring at -40°C pieces of metallic sodium until the colour of the solution changes permanently to blue. The ammonia is evaporated and ethanol is added. The mixture is filtered and the

filtrate evaporated to dryness. The residue is crystallized from ethyl acetate to give a product melting at 139-142°C. The compound is converted to the hydrochloride e.g. in a mixture of ethyl acetate and isopropanol by adding HCl-ethyl acetate; m.p. 201-203°C.

$^1$H-NMR (base): 2.4 (s, 6H), 3.3 (d, 2H), 4.8 (t, 1H), 6.7 (s, 2H), 6.95 (s, 3H), 7.5 (broad peak, 1H)

c) 2-[2-(2,6-dimethylphenyl)-ethenyl]-imidazole

10 g 2-[2-(2,6-dimethylphenyl)-1-hydroxyethyl]-imidazole and 50 g potassiumhydrogensulfate are combined. The agents are mixed well and the mixture heated to 150°C and stirred now and then for 3 hours. The mixture is cooled and dissolved in ethanol. The solution is filtered and evaporated to dryness. The obtained crude 2-[2-(2,6-dimethylphenyl)-ethenyl]-imidazole is used without purification in the next step d). [The hydrochloride of the pure product (trans-form) has an m.p. of 235-240°C].

d) 2-[2-(2,6-dimethylphenyl)-ethyl]-imidazole

10 g crude 2-[2-(2,6-dimethylphenyl)-ethenyl]-imidazole is dissolved in 50 ml ethanol and then is hydrogenated at about 60°C at normal pressure until the theoretical amount of hydrogen has been consumed. The mixture is filtered and the filtrate evaporated to dryness. The residue is crystallized from ethyl acetate,

m.p. 160-162°C. The product can be converted to the hydrochloride in isopropanol with HCl-ethylacetate, m.p. 205-209°C.

$^{1}$H-NMR (HCl-salt): 2.38 (s, 6H), 2.69 (t, 2H), 3.06 (t, 2H), 3.97 (s, 4H), 4.81 (s, 2H), 7.16 (s, 3H)

$^{13}$H-NMR (HCl-salt): 21.00 (q), 27.064 (t), 29.425 (t), 121.059 (d), 129.414 (d), 130.807 (d), 137.951 (s), 139.374 (s), 149.031 (s).

Using the procedure of Example 1 the following compounds were prepared:

1-Benzyl-2-[2-(3,4-dimethylphenyl)-1-hydroxyethyl]-imidazole

M.p. (base): 133-140°C (from ethyl acetate)

1-Benzyl-2-[3-(2,6-dimethylphenyl)-1-hydroxypropyl]-imidazole

M.p. (base): 146-148°C (from ethyl acetate)

1-Benzyl-2-[α-(2,6-dimethylphenyl)-hydroxymethyl]-imidazole

M.p. (base): 120-122°C (from water-ethanol)

M.p. (hydrochloride): 205-209°C

1-Benzyl-2-[α-(3-methoxyphenyl)-hydroxymethyl]-imidazole

M.p. (base): 114-116°C.

M.p. (hydrochloride): 155-158°C.

1-Benzyl-2-[α-(2,3-dimethylphenyl)-hydroxymethyl]-imidazole

M.p. (base): 108-110°C.

2-[2-(2,3-dimethylphenyl)-1-hydroxyethyl]-imidazole

M.p. (base): 142-148°C

1-Benzyl-2-[2-(2-methylphenyl)-1-hydroxyethyl)]-imidazole

M.p. (base): 135-137°C (from ethyl acetate)

2-[2-(2-methylphenyl)-1-hydroxyethyl]-imidazole

M.p. (base): 173-175°C (from water)

2-[2-(2-methylphenyl)-ethenyl]-imidazole

M.p. (base): 165-169°C (from water)

2-[2-(2-methylphenyl)-ethyl]-imidazole

M.p. (hydrochloride): 171-173°C

1-Benzyl-2-[2-(4-methylphenyl)-1-hydroxyethyl]-imidazole

M.p. (base): 125-127°C (from ethyl acetate)

2-[2-(4-methylphenyl)-1-hydroxyethyl]-imidazole

M.p. (base): 208-209°C (from water)

2-[2-(4-methylphenyl)-ethenyl]-imidazole

M.p. (base): 177-183°C (from ethyl acetate)

2-[2-(4-methylphenyl)-ethyl]-imidazole

M.p. (base): 136-138°C

2-[3-(2,6-dimethylphenyl)-1-hydroxypropyl]-imidazole

M.p. (base): 178-181°C

2-[2-(2,5-dimethylphenyl)-1-hydroxyethyl]-imidazole

M.p. (base): 174-176°C (from ethyl acetate)

2-[2-(3,4-dimethylphenyl)-1-hydroxyethyl]-imidazole

M.p. (base): 135-137°C (from water)

2-[2-(3-methylphenyl)-1-hydroxyethyl]-imidazole

M.p. (base): 178-181°C (from ethyl acetate)

2-[α-(2,6-dimethylphenyl)-hydroxymethyl]-imidazole

M.p. (hydrochloride): 235-237°C

2-[2-(2,3-dimethylphenyl)-ethyl]-imidazole

M.p. (hydrochloride): 190-192°C

2-[2-(2,3-dimethylphenyl)-1-hydroxyethyl]-imidazole

2-(3-phenyl-1-hydroxypropyl)-imidazole

M.p. (base): 140-144°C (from water)

2-[2-(3,4-dimethylphenyl)-ethenyl]-imidazole

M.p. (base): 154-161°C, m.p. (hydrochloride): 247-250°C

2-[5-(2,6-dimethylphenyl)-1-pentenyl]-imidazole

2-[2-(2,6-dimethylphenyl)-ethyl]-4-(2,6-dimethylbenzyl)-imidazole

M.p. (base): 183-186°C (from acetonitrile)

2-[2-(2,5-dimethylphenyl)-ethenyl]-imidazole

M.p. (hydrochloride): 228-234°C.

2-[3-(2,6-dimethylphenyl)-propyl]-imidazole

$^{13}$C-NMR: 19.556 (q), 28.274 (t), 28.728 (t), 29.092 (t), 121.150 (d), 125.630 (d), 128.022 (d), 135.802 (s), 138.345 (s).

1-Benzyl-2-[4-(2,6-dimethylphenyl)-1-hydroxybutyl]-imidazole

M.p. (base): 164-166°C (from ethyl acetate)

2-[4-(2,6-dimethylphenyl)-1-hydroxybutyl]-imidazole

M.p. (base): 135-140°C (from water)

2-[5-(2,6-dimethylphenyl)-1-hydroxypentyl]-imidazole

M.p. (base): 145-148°C.

M.p. (hydrochloride): 110-111°C

$^{13}$C-NMR: 19.919 (a), 26.821 (t), 30.091 (t), 30.484 (t), 37.689 (t), 68.930 (d), 122.270 (d), 126.327 (d), 128.900 (d), 136.619 (s), 140.191 (s), 152.270 (s)

2-(2-phenylethyl)-imidazole

M.p. (base): 79.81°C. M.p. (hydrochloride): 124-126°C.

2-[2-(3,4-dimethylphenyl)-ethyl]-imidazole

M.p. (base): 92-94°C (from water).

2-(2,6-dimethylbenzyl)-imidazole

M.p. (base): 232-234°C. M.p. (hydrochloride): 244-249°C.

2-(2,3-dimethylbenzyl)-imidazole

M.p. (base): 153-155°C (from aqueous ethanol)

2-[2-(2,6-dimethylphenyl)-ethyl]-1-methyl-imidazole

M.p. (hydrochloride): 197-199°C.

<u>Example 2</u>

2-[2-(2,6-dimethylphenyl)-ethyl]-imidazole

a) 1-Benzyl-2-[2-(2,6-dimethylphenyl)-ethenyl]-imidazole

59 g 2,6-dimethylbenzyl-triphenylphosphoniumchloride are dissolved in 300 ml tetrahydrofuran. The solution is heated to about 40°C and at this temperature in a nitrogen atmosphere 9.5 g butyl lithium in hexane is then added during 1-2 hours. After the addition, the mixture is stirred for 2 hours at about 60°C, and then cooled to 20°C. 21.2 g N-benzyl-2-imidazole-aldehyde in 100 ml tetrahydrofuran is added dropwise to the reaction mixture. After the addition, the mixture is refluxed for 4 hours, cooled and

water is added. The solution is acidified with hydrochloric acid and washed with ethyl acetate. The aqueous solution is then extracted with methylene chloride, the extract is washed with water, diluted sodium hydroxide solution and again with water and finally evaporated to dryness. The residue containing crude product can be used without purification in the next step.

b) 1-Benzyl-2-[2-(2,6-dimethylphenyl)-ethyl]-imidazole

11.7 g of 1-benzyl-2-[2-(2,6-dimethylphenyl)-ethenyl]-imidazole from the preceding step is dissolved in 50 ml 95% ethanol. 10% Pd on carbon is added as catalyst and the mixture is stirred in hydrogen atmosphere until the calculated amount of hydrogen is consumed. The mixture is then filtered and evaporated to dryness. The crude 1-benzyl-2-[2-(2,6-dimethylphenyl)-ethyl]-imidazole thus obtained is directly used in the next step.

c) 2-[2-(2,6-dimethylphenyl)-ethyl]-imidazole

11g of 1-benzyl-2-[2-(2,6-dimethylphenyl)-ethyl]-imidazole is suspended in a mixture of 20 ml liquid ammonia and 30 ml ether cooled to -40°C. Metallic sodium is added in small pieces until the colour of the solution has changed permanently to blueish growing at the same time considerably darker. After the addition the stirring at -40°C is continued for 15 minutes. The ammonia is

evaporated and ethanol is carefully added and then water. The product is extracted with methylene chloride, evaporated to dryness and the residue crystallized from ethyl acetate. The melting point of the obtained 2-[2-(2,6-dimethylphenyl)-ethyl]-imidazole is 160-164°C.

Example 3

2-[2-(2,6-dimethylphenyl)-ethyl]-4-methyl-imidazole

a) 3-(2,6-dimethylphenyl)-propanimidamide hydrochloride

18.5 g 3-(2,6-dimethylphenyl)-propionitrile and 5.5 g absolute ethanol are combined. The mixture is cooled in an ice-bath and dry HCl-gas is passed therein at dry conditions and the mixture is kept for 5 days at 0°C. Another 5 ml of ethanol is added and then by stirring 29 ml 9% ethanolic ammonia solution. Thereafter is stirred for 3 hours, filtered, evaporated to a small volume and cooled. The precipitate, which is the desired product, is collected by filtration, washed with cold absolute ethanol and dried over conc. $H_2SO_4$, m.p. 215-219°C.

b) 3 g of the imidamide derivative of step a) and 1.1 g hydroxyacetone are combined in 7 ml of liquid ammonia in an autoclave. The autoclave is closed and the reaction mixture is stirred at 80°C for one day. Then it is cooled, the ammonia is evaporated and the residue suspended in 10% hydrochloric acid. The mixture is

heated for 30 minutes at about 50°C and the pH adjusted to 4-5. The mixture is washed with toluene and the aqueous phase made alkaline with sodium hydroxide. The precipitate containing the desired product is filtered and washed with water. The dried product is converted to the hydrochloride in ethyl acetate by adding HCl-isopropanol. The m.p. of the hydrochloride is 184-186°C.

$^{1}$H-NMR (HCl-salt): 2.30 (s, 6H), 2.40 (s, 3H), 3.12 (s, 4H), 4.90 (s, 2H), 7.13 (s, 1H), 7.15 (s, 3H)

$^{13}$C-NMR (HCl-salt): 11.292 (q), 21.039 (q), 26.912 (t), 29.394 (t), 117.154 (d), 129.142 (d), 130.595 (d), 131.503 (s), 137.830 (s), 139.102 (s), 147.759 (s)

Using the procedure of Example 3 the following compounds were prepared:

2-(2,6-dimethylbenzyl)-4,5-dimethyl-imidazole
M.p. (hydrochloride): 268-270°C

2-(2,6-dimethylbenzyl)-4-hydroxymethyl-imidazole
M.p. (hydrochloride): 184-185°C

2-(2,6-dimethylbenzyl)-4-methyl-imidazole
M.p. (hydrochloride): 225-227°C (from acetone)

2-(2-methylbenzyl)-4,5-dimethyl-imidazole
M.p. (base): 162-164°C (from water-methanol)

2-(2-methylbenzyl)-4-hydroxymethyl-imidazole
M.p. (base): 146-148°C (from water-methanol)

2-(2-methylbenzyl)-4-methyl-imidazole
M.p. (hydrochloride): 110-112°C (from acetone)

2-[(2,3-dimethylphenyl)-ethyl]-4-hydroxymethyl-imidazole
M.p. (base): 170-173°C

2-[2-(2,6-dimethylphenyl)-ethyl]-4-hydroxymethyl-imidazole

M.p. (hydrochloride): 148-150°C

2-[2-(2,3-dimethylphenyl)-ethyl]-4-methyl-imidazole

M.p. (hydrochloride): 124-126°C (from water)

2-[2-(2,6-dimethylphenyl)-ethyl]-4,5-dimethyl-imidazole

M.p. (hydrochloride): 234-237°C

2-[2-(2,6-dimethylphenyl)-ethyl]-4-ethyl-imidazole

M.p. (hydrochloride): 145-149°C

2-[2-(2,3-dimethylphenyl)-ethyl]-4-ethyl-imidazole

2-[2-(2,3-dimethylphenyl)-ethyl]-4-methyl-imidazole

M.p. (hydrochloride): 124-127°C

2-[2-(2,3-dimethylphenyl)-ethyl]-4,5-dimethyl-imidazole

M.p. (base): 138-145°C

2-[2-(2-methylphenyl)-ethyl]-4-methyl-imidazole

2-[2-(2-chlorophenyl)-ethyl]-4-methyl-imidazole

2-[2-(2,6-dichlorophenyl)-ethyl]-4-methyl-imidazole

2-[2-(4-chlorophenyl)-ethyl]-4-methyl-imidazole

2-[3-(2,4-dimethylphenyl)-propyl]-4-methyl-imidazole

2-[3-(2,3-dimethylphenyl)-propyl]-4-ethyl-imidazole

2-[3-(2,6-dimethylphenyl)-propyl]-4-methyl-imidazole

2-[3-(2,4-dichlorophenyl)-propyl]-4-methyl-imidazole

2-[2-(4-ethylphenyl)-ethyl]-4-methyl-imidazole

2-[2-(3,4-dihydroxyphenyl)-ethyl]-4-methyl-imidazole

2-(3,4-dimethoxybenzyl)-4-methyl-imidazole

2-(3,4-dihydroxybenzyl)-4-methyl-imidazole

2-(3-methoxybenzyl)-4- methyl-imidazole

2-(3-hydroxybenzyl)-4-methyl-imidazole

2-(2,6-dimethylbenzyl)-4-ethyl-imidazole

M.p. (hydrochloride): 198-203°C

2-(2,3-dimethylbenzyl)-4-methyl-imidazole

M.p. (base): 135-137°C

Example 4

2-[2-(2,3-dimethylphenyl)-ethyl]-imidazole

a) 2-[2-(2,3-dimethylphenyl)-ethyl]-imidazoline

24 g 3-(2,3-dimethylphenyl)-propionitrile and 65.2 g ethylenediamine p-toluenesulfonate salt are combined and the mixture is heated to 170°C for 30 hours and stirred intermittently. The mixture is cooled and suspended in ethyl acetate. The ethyl acetate is decanted and the residue dissolved in water and the solution extracted with methylene chloride. The methylene chloride extract is washed with water, diluted sodium hydroxide solution and again with water. Then it is evaporated to dryness. The obtained product is converted to the hydrochloride in isopropanol with HCl-ethyl acetate, m.p. 205-208°C.

$^1$H-NMR (HCl-salt): 2.29 (s, 3H), 2.35 (s, 3H), 2.95 (m, 4H), 3.93 (s, 4H), 4.84 (s, 2H), 7.18 (s, 3H)

b) 2-[2-(2,3-dimethylphenyl)-ethyl]-imidazole

2 g of the above obtained 2-[2-(2,3-dimethylphenyl)-ethyl]-2-imidazoline, 30 g barium manganate and 500 ml distilled methylene chloride are refluxed for 2 days. The mixture is cooled and 10 g dry magnesium sulfate is added. Then is filtered and the filtrate evaporated to dryness. In this way a pure product is obtained, which then can be converted to the hydrochloride in isopropanol-ethylacetate with HCl-ethyl acetate,

m.p. 188-192°C. M.p. (base): 115-117°C.

$^{13}C$-NMR: 16.589 (q), 22.311 (q), 28.910 (t), 33.633 (t), 121.029 (d), 129.476 (d), 129.566 (d), 131.140 (d), 137.739 (s), 139.707 (s), 140.555 (s), 149.303 (s)

Following the procedure of Example 4 the following compounds were prepared:

2-[2-(2,6-dimethylphenyl)-ethyl]-imidazoline

M.p. (hydrochloride): 175-178°C.

2-[3-(2,6-dimethylphenyl)-propyl]-imidazoline

M.p. (hydrochloride): 200-204°C.

CLAIMS

1. Substituted imidazoles and imidazolines of the general formula

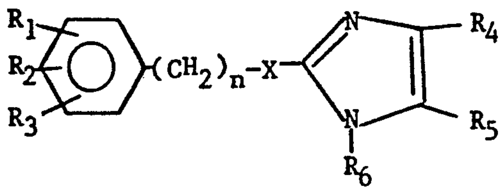 or 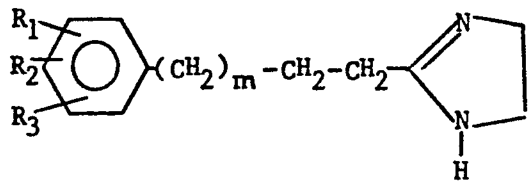

(I) (II)

wherein each of $R_1$, $R_2$ and $R_3$, which can be the same or different, is hydrogen, chloro, bromo, fluoro, methyl, ethyl, methoxy, amino, hydroxy or nitro; $R_4$ and $R_5$, which can be the same or different, are hydrogen, an alkyl radical of 1 to 7 carbon atoms, hydroxymethyl or

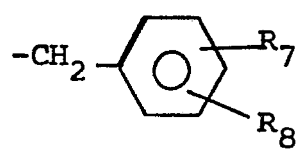 ;

$R_6$ is hydrogen, an alkyl group of 1 to 7 carbon atoms or

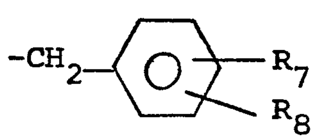 ;

$R_7$ and $R_8$ are hydrogen, chloro or methyl; X is -CH=CH- or $-CHR_9-$; $R_9$ is hydrogen or hydroxy; n is 0-7; and m is 0-6;

provided that, in general formula (I):

a) when n = 0 and $R_1$ and $R_2$ are both hydrogen then $R_3$ is not hydrogen, methoxy or 4-chloro,

b) when n = 0 and X = -CHOH-, then $R_6$ is not methyl,

c) when n = 0, X = -CH=CH-, and $R_1$ and $R_2$ are hydrogen, $R_3$ is not nitro, amino or hydroxy,

d) when n is 1 and $R_1$, $R_2$, $R_3$ and $R_5$ are all hydrogen, then if X is $CH_2$ and $R_6$ is hydrogen, $R_4$ is other than hydroxymethyl or if X is CHOH and $R_4$ is hydrogen, $R_6$ is other than methyl, and

e) when n is 2 and X is $CH_2$ then $R_1$, $R_2$, $R_3$, $R_4$, $R_5$ and $R_6$ are not all hydrogen, and

f) in general formula (II),

when m is 0 or 1, $R_1$, $R_2$ and $R_3$ are not all hydrogen; and their non-toxic, pharmaceutically acceptable acid addition salts.

2. Substituted imidazoles according to Claim 1 wherein X is $-CH_2-$.

3. Substituted imidazoles according to Claim 1 wherein X is -CHOH-.

4. Substituted imidazoles according to Claim 1 wherein X is -CH=CH-.

5. Substituted imidazolines according to Claim 1 of the general formula

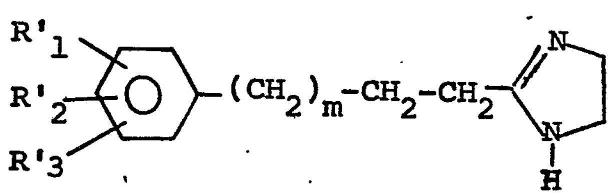

wherein $R'_1$, $R'_2$ and $R'_3$ are each hydrogen, chloro, bromo, fluoro or methyl and m is as defined in claim 1.

6. Substituted imidazoles according to Claim 2 wherein $R_6$ is hydrogen.

7. Substituted imidazoles according to Claim 2 or 6 wherein $R_4$ and $R_5$ are both hydrogen.

8. Substituted imidazoles according to Claim 3 wherein $R_6$ is hydrogen.

9. Substituted imidazoles according to Claim 3 or 8 wherein $R_4$ and $R_5$ are both hydrogen.

10. Substituted imidazoles according to Claim 9 wherein $R_1$, $R_2$ and $R_3$ are hydrogen, chloro, bromo, fluoro, or methyl and n is 2-7.

11. Substituted imidazoles according to Claim 4 wherein $R_6$ is hydrogen.

12. Substituted imidazoles according to Claim 4 or 11 wherein $R_4$ and $R_5$ are both hydrogen.

13. Substituted imidazoles according to Claim 1 wherein each of $R_1$, $R_2$ and $R_3$ is hydrogen, chloro, bromo, fluoro or methyl, X is $-CH_2-$, each of $R_4$ and $R_5$ is hydrogen or alkyl of 1 to 4 carbon atoms, $R_6$ is hydrogen, and n is 1-7.

14. Substituted imidazoles according to Claim 1 or 13, wherein $R_6$ is hydrogen, X is $-CH_2-$ and n is 1.

15. Substituted imidazoles according to Claim 11 wherein each of $R_1$, $R_2$ and $R_3$ is hydrogen, chloro,

bromo, fluoro or methyl, and each of $R_4$ and $R_5$ is hydrogen or alkyl of 1 to 4 carbon atoms.

16. Substituted imidazoles according to Claim 1 wherein $R_1$ and $R_2$ are both methyl, $R_3$ is hydrogen, n is 0, 1 or 2, X is $CH_2$, CHOH or, when n is 0 CH=CH, $R_4$ is hydrogen or methyl, one of $R_5$ and $R_6$ is hydrogen or benzyl and the other is hydrogen.

17. 2-[2-(2,6-Dimethylphenyl)-ethyl]-imidazole and its non-toxic pharmaceutically acceptable acid addition salts.

18. 2-[2-(2,6-Dimethylphenyl)-1-hydroxyethyl]-imidazole and its non-toxic pharmaceutically acceptable acid addition salts.

19. 2-[2-(2,6-Dimethylphenyl)-ethenyl]-imidazole and its non-toxic pharmaceutically acceptable acid addition salts.

20. 2-[2-(2,5-Dimethylphenyl)-1-hydroxyethyl]-imidazole and its non-toxic pharmaceutically acceptable acid addition salts.

21. 2-[2-(3,4-Dimethylphenyl)-1-hydroxyethyl]-imidazole and its non-toxic pharmaceutically acceptable acid addition salts.

22. 2-[2-(2,3-Dimethylphenyl)-ethyl]-4-methyl-imidazole and its non-toxic pharmaceutically acceptable acid addition salts.

23. 2-[2-(3,4-Dimethylphenyl)-ethyl]-imidazole and its non-toxic pharmaceutically acceptable acid

addition salts.

24. 2-[2-(2,6-Dimethylphenyl)-ethyl]-4-methyl-imidazole and its non-toxic pharmaceutically acceptable acid addition salts.

25. 2-(2-Methylbenzyl)-4,5-dimethyl-imidazole and its non-toxic pharmaceutically acceptable acid addition salts.

26. 2-[2-(2,3-Dimethylphenyl)-ethyl]-imidazoline and its non-toxic pharmaceutically acceptable acid addition salts.

27. 2-[2-(2,6-Dimethylphenyl)-ethyl]-imidazoline and its non-toxic pharmaceutically acceptable acid addition salts.

28. 2-[2-(2,5-Dimethylphenyl)-ethyl]-imidazole and its non-toxic pharmaceutically acceptable acid addition salts.

29. 1-Benzyl-2-[3-(2,6-dimethylphenyl)-1-hydroxypropyl]-imidazole and its non-toxic pharmaceutically acceptable acid addition salts.

30. A process for the preparation of a compound as claimed in Claim 1, which comprises

a) reacting an imidazole aldehyde of the formula

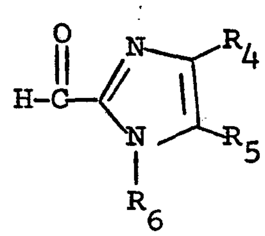

wherein $R_4$ and $R_5$ are defined as in Claim 1 and $R_6$, which can also be in the 3-position of the imidazole ring, is hydrogen or an alkyl group with 1 to 7 carbon atoms with an aralkylmagnesiumhalide of the formula:

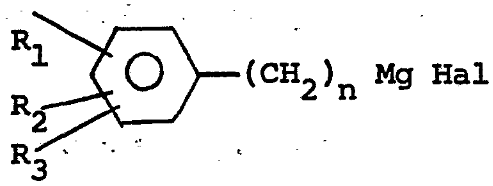

wherein $R_1$, $R_2$, $R_3$ and n are defined as in Claim 1 and Hal is a halogen atom to give a compound of the formula

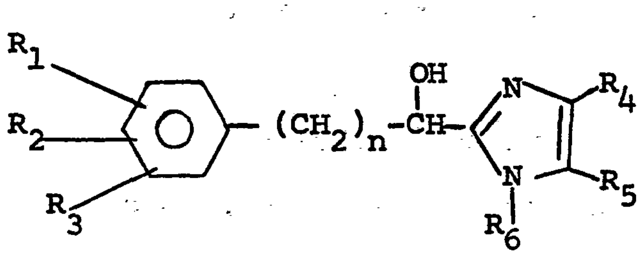

wherein $R_1$, $R_2$, $R_3$, $R_4$, $R_5$, $R_6$ and n are defined as above which

b) is dehydrated to a compound of the formula:

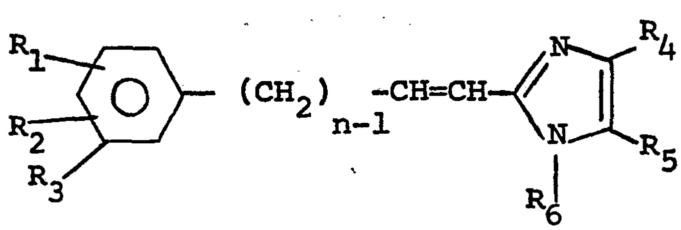

wherein $R_1$, $R_2$, $R_3$, $R_4$, $R_5$, $R_6$ and n are defined as above which.

c) is hydrogenated to a compound of formula (I) as defined in claim 1 wherein X is $-CH_2-$ and $R_6$ is hydrogen or an alkyl group of 1 to 7 carbon atoms.

31. A process for the preparation of a compound as claimed in claim 1, which comprises

a) reacting an imidazole aldehyde of the formula

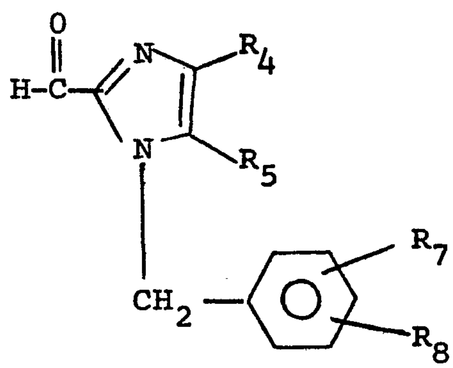

wherein $R_4$, $R_5$, $R_7$ and $R_8$ are as defined in claim 1, and wherein the N-substituent also can be in the 3-position of the imidazole ring, with an aralkylmagnesium halide of the formula:

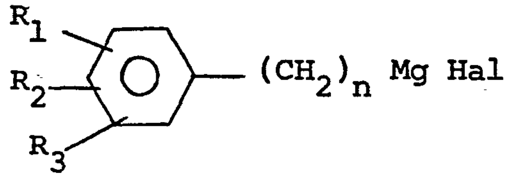

wherein $R_1$, $R_2$, $R_3$ and n are defined as in claim 1 and Hal is a halogen atom to give a compound of the formula

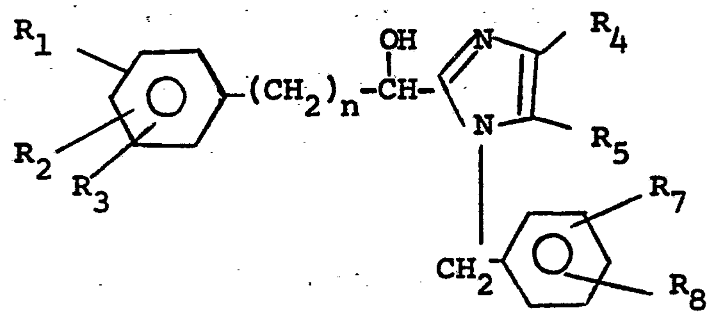

wherein $R_1$, $R_2$, $R_3$, $R_4$, $R_5$, $R_7$, $R_8$ and n are as defined above which

b) is reacted with sodium in liquid ammonia at a low temperature, to form a compound of the formula:

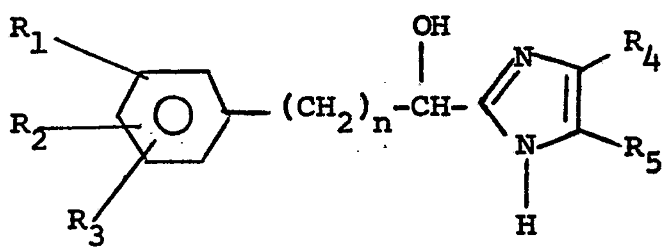

wherein $R_1$, $R_2$, $R_3$, $R_4$, $R_5$ and n are as defined above which

c) is dehydrated to a compound of the formula

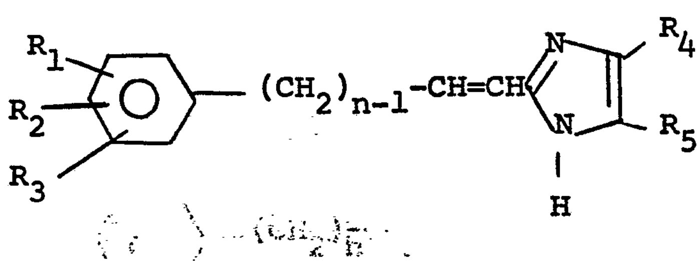

wherein $R_1$, $R_2$, $R_3$, $R_4$, $R_5$ and n are as defined above which

d) is hydrogenated to a compound of formula (I) as defined in claim 1 wherein X is $-CH_2-$ and $R_6$ is hydrogen.

32. A process for the preparation of a compound as claimed in claim 1 which comprises

a) reacting an imidazole aldehyde of the formula:

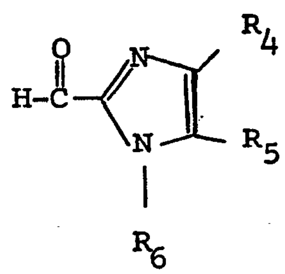

wherein $R_4$, $R_5$ and $R_6$, which can also be in the 3-position of the imidazole ring, are as defined in claim 1 with butyl lithium and an aralkyltriphenylphosphonium halide of the formula

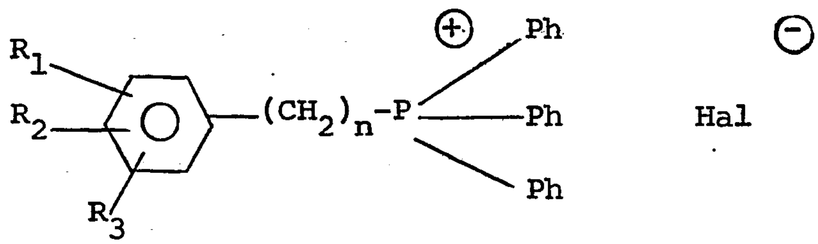

wherein $R_1$, $R_2$, $R_3$ and n are as defined in claim 1, Ph is a phenyl group and Hal is a halogen atom to give a compound of the formula

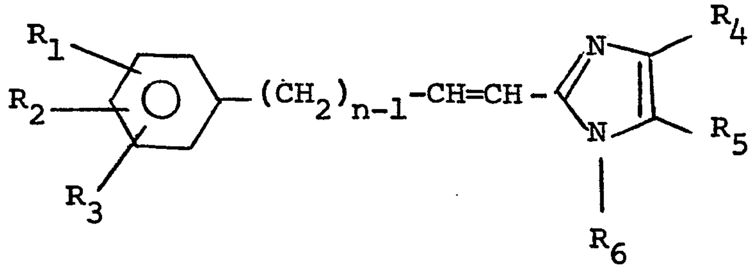

wherein $R_1$, $R_2$, $R_3$, $R_4$, $R_5$, $R_6$ and n are as defined in claim 1, which is

b) hydrogenated to give a compound of the formula:

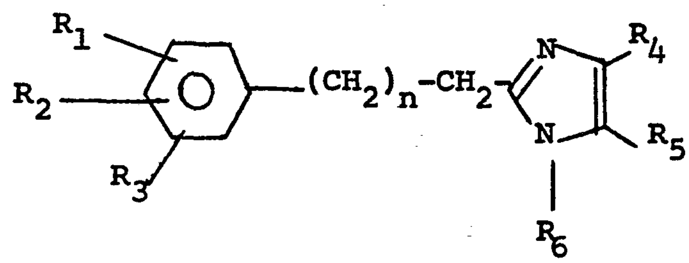

wherein $R_1$, $R_2$, $R_3$, $R_4$, $R_5$, $R_6$ and n are as defined above which when

$R_6$ is 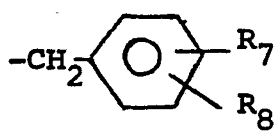 is

c) reacted with sodium in liquid ammonia to give a compound of formula (I) as defined in claim 1 wherein X is $-CH_2-$ and $R_6$ is hydrogen.

33. A process for the preparation of a compound as claimed in claim 1, which comprises reacting an aralkylimidamide of the formula:

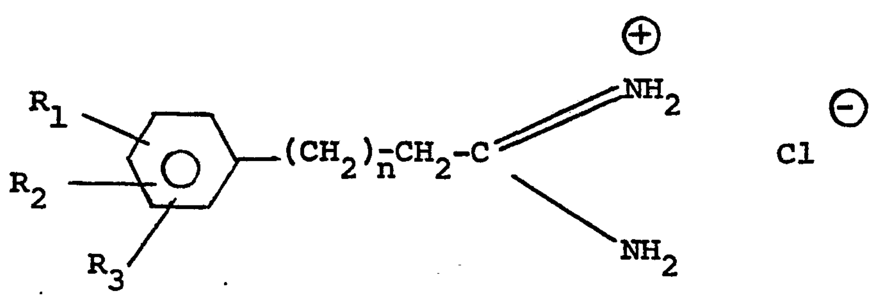

wherein $R_1$, $R_2$, $R_3$ and n are as defined in claim 1 with a compound of the formula

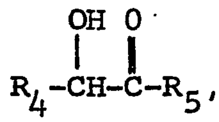, 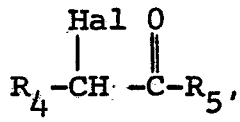, 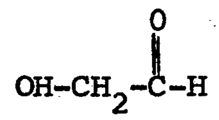 or

$$HO-CH_2-CH_2-CH(O\ Alkyl)_2$$

to form a compound of formula (I) as defined in claim 1, wherein X is $-CH_2-$.

34. A process for the preparation of a compound as claimed in claim 1, which comprises hydrolysing a compound of the formula:

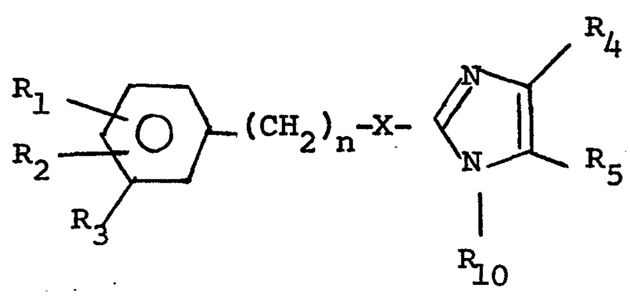

wherein $R_1$, $R_2$, $R_3$, $R_4$, $R_5$, X and n are as defined in claim 1 and $R_{10}$, which also can be in the 3-position of the imidazole ring, is a hydrolysable group to give a compound of the formula:

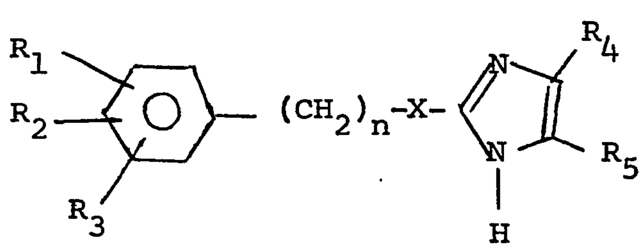

wherein $R_1$, $R_2$, $R_3$, $R_4$, $R_5$, X and n are as defined in claim 1.

35. A process for the preparation of a compound as claimed in claim 1 which comprises

a) reacting an aralkylnitrile of the formula:

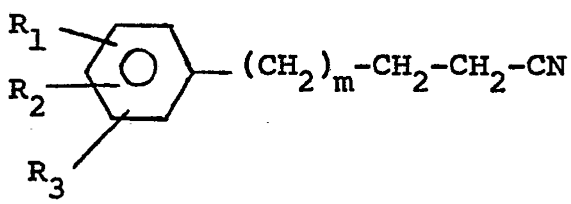

wherein $R_1$, $R_2$, $R_3$ and m are as defined in claim 1 with a salt of ethylenediamine to give a compound of the formula:

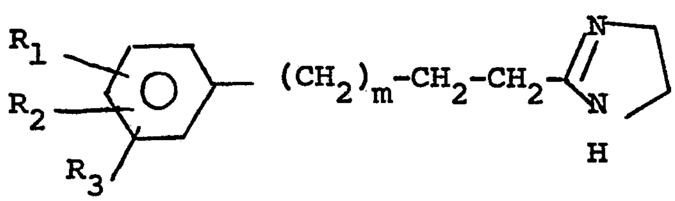

wherein $R_1$, $R_2$, $R_3$ and m are as defined above, which is

b) aromatised to give a compound of formula (I), wherein $R_4$ and $R_5$ are hydrogen.

36. A process for the preparation of a compound as claimed in claim 1, which comprises reacting an iminoether of the formula

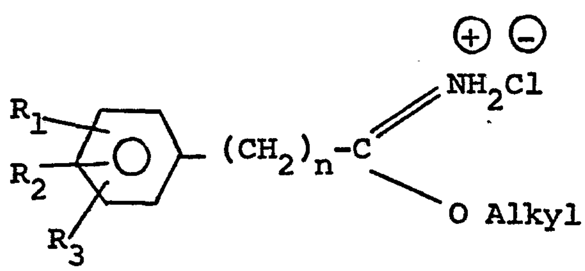

with an aminoketone, aminoacetaldehyde or its acetal of the formula:

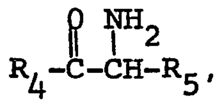, 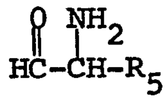 or 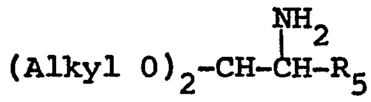

wherein $R_1$, $R_2$, $R_3$, $R_4$, $R_5$ and n are the same as before to give a compound of formula I as defined in claim 1, wherein X is $-CH_2-$.

37. A pharmaceutical composition comprising a compound as claimed in any of claims 1 to 29 in association with a compatible pharmaceutically acceptable carrier.

38. A substituted imidazole or imidazoline of the general formula:

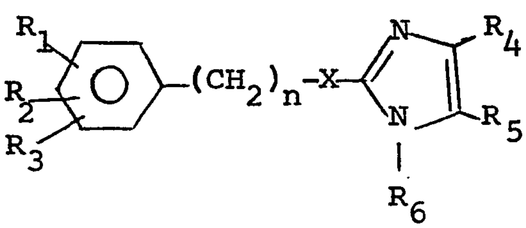 or 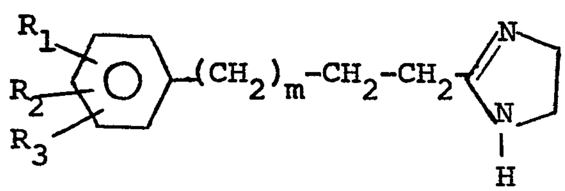

(I) (II)

wherein each of $R_1$, $R_2$ and $R_3$, which can be the same or different, is hydrogen, chloro, bromo, fluoro, methyl, ethyl, methoxy, amino, hydroxy or nitro; $R_4$ and $R_5$, which can be the same or different, are hydrogen, an alkyl radical of 1 to 7 carbon atoms, hydroxymethyl or

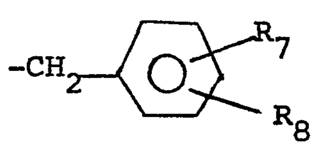 ;

$R_6$ is hydrogen, an alkyl group of 1 to 7 carbon atoms or

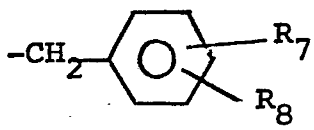 ;

$R_7$ and $R_8$ are hydrogen, chloro or methyl; X is $-CH=CH-$ or $-CHR_9-$; $R_9$ is hydrogen or hydroxy; n is 0-7; and m is 0-6;
and their non-toxic pharmaceutically acceptable acid addition salts, for use in therapy in the treatment of hypertension and thrombosis.

39. A substituted imidazole or imidazoline as claimed in any of claims 16 to 29 for use in therapy in the treatment of hypertension and thrombosis.

0064820

C L A I M S

1. A process for the preparation of therapeutically active substituted imidazoles and imidazolines of the general formula

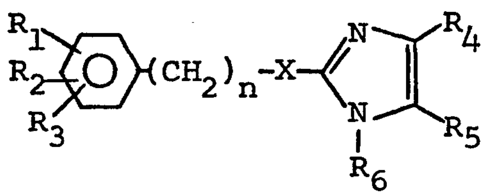

or

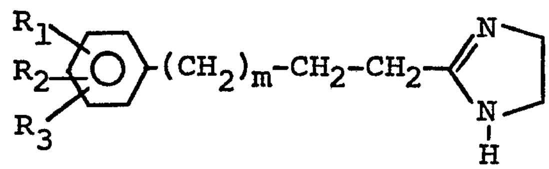

(I)    (II)

wherein each of $R_1$, $R_2$ and $R_3$, which can be the same or different, is hydrogen, chloro, bromo, fluoro, methyl, ethyl, methoxy, amino, hydroxy or nitro; $R_4$ and $R_5$, which can be the same or different, are hydrogen, an alkyl radical of 1 to 7 carbon atoms, hydroxymethyl or

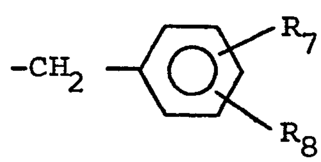

;

$R_6$ is hydrogen, an alkyl group of 1 to 7 carbon atoms or

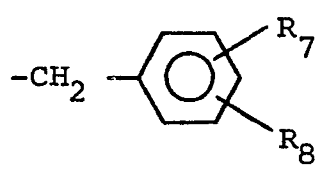

;

$R_7$ and $R_8$ are hydrogen, chloro or methyl; X is -CH=CH- or $-CHR_9-$; $R_9$ is hydrogen or hydroxy; n is 0-7; and m is 0-6;

provided that, in general formula (I):

a) when n = 0 and $R_1$ and $R_2$ are both hydrogen then $R_3$ is not hydrogen, methoxy or 4-chloro

b) when n = 0 and X = -CHOH-, then $R_6$ is not methyl

c) when n = 0, X = -CH=CH-, and $R_1$ and $R_2$ are hydrogen, $R_3$ is not nitro, amino, or hydroxy

d) when n is 1 and $R_1$, $R_2$, $R_3$ and $R_5$ are all hydrogen then if X is $CH_2$ and $R_6$ is hydrogen, $R_4$ is other than hydroxymethyl or if X is CHOH and $R_4$ is hydrogen, $R_6$ is other than methyl, and

e) when n is 2 and is $CH_2$ then $R_1$, $R_2$, $R_3$, $R_4$, $R_5$ and $R_6$ are not all hydrogen, and

f) in general formula (II), when m is 0 or 1, $R_1$, $R_2$ and $R_3$ are not all hydrogen, and their non-toxic, pharmaceutically acceptable acid addition salts;

which comprises

a) reacting an imidazole aldehyde of the formula

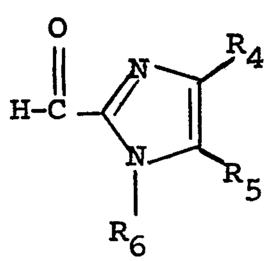

wherein $R_4$ and $R_5$ are as hereinbefore defined and $R_6$, which can also be in the 3-position of the imidazole ring, is hydrogen or an alkyl group with 1 to 7 carbon atoms

with an aralkylmagnesiumhalide of the formula:

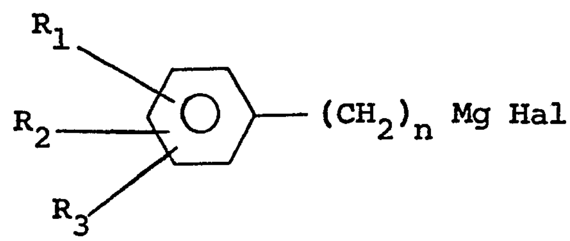

wherein $R_1$, $R_2$, $R_3$ and n are as hereinbefore defined and Hal is a halogen atom, to give a compound of the formula

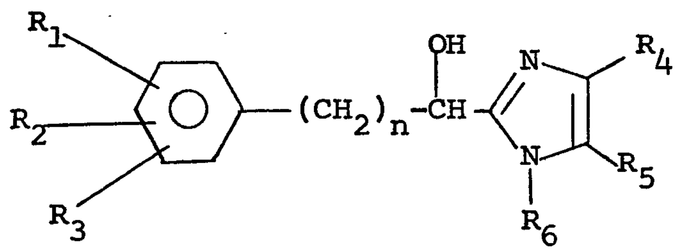

wherein $R_1$, $R_2$, $R_3$, $R_4$, $R_5$, $R_6$ and n are defined as above which

is dehydrated to a compound of the formula:

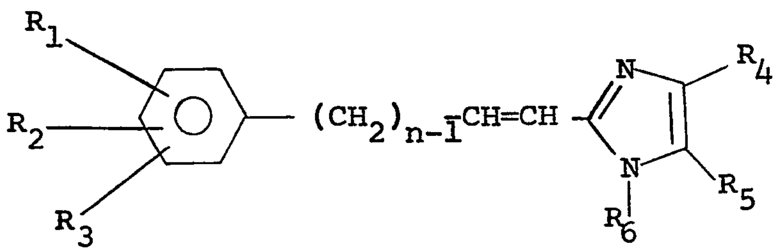

wherein $R_1$, $R_2$, $R_3$, $R_4$, $R_5$, $R_6$ and n are defined as above which

is hydrogenated to a compound of formula (I) wherein X is $-CH_2-$ and $R_6$ is hydrogen or an alkyl group of 1 to 7 carbon atoms,

b) reacting an imidazole aldehyde of the formula

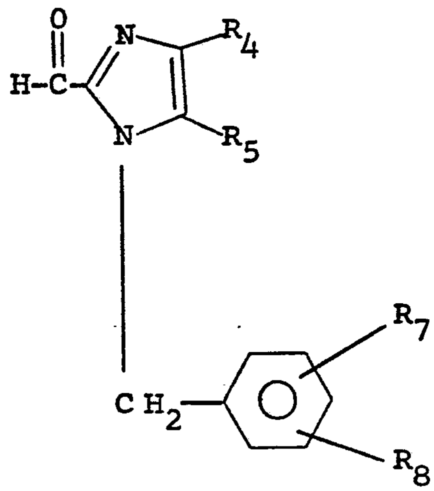

wherein $R_4$, $R_5$, $R_7$ and $R_8$ are as hereinbefore defined, and wherein the N-substituent also can be in the 3-position of the imidazole ring, with an aralkylmagnesium halide of the formula:

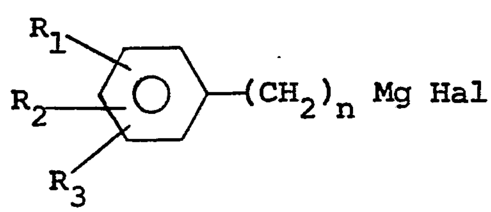

wherein $R_1$, $R_2$, $R_3$ and n are as hereinbefore defined and Hal is a halogen atom to give a compound of the formula

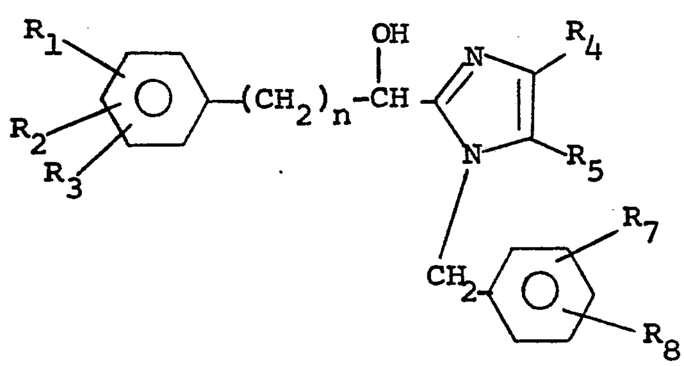

wherein $R_1$, $R_2$, $R_3$, $R_4$, $R_5$, $R_7$, $R_8$ and n are as defined above which

is reacted with sodium in liquid ammonia at a low temperature, to form a compound of the formula:

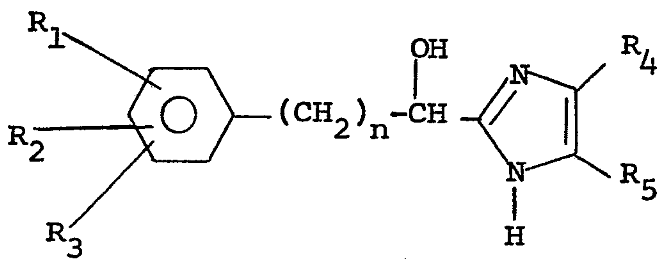

wherein $R_1$, $R_2$, $R_3$, $R_4$, $R_5$ and n are as defined above which

is dehydrated to a compound of the formula

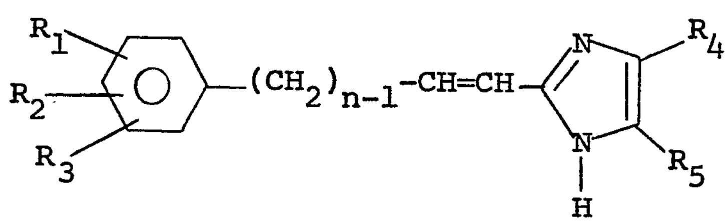

wherein $R_1$, $R_2$, $R_3$, $R_4$, $R_5$ and n are as defined above which

is hydrogenated to a compound of formula (I) wherein X is $-CH_2-$ and $R_6$ is hydrogen,

c) reacting an imidazole aldehyde of the formula:

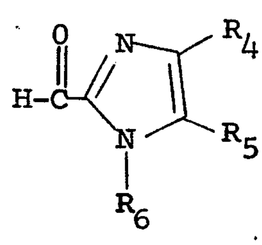

wherein $R_4$, $R_5$ and $R_6$, which can also be in the 3-position of the imidazole ring, are as defined above with butyl lithium and an aralkyltriphenylphosphonium halide of the formula

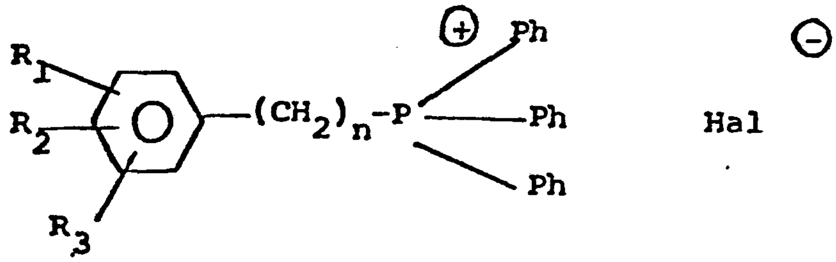

wherein $R_1$, $R_2$, $R_3$ and n are as defined above, Ph is a phenyl group and Hal is a halogen atom to give a compound of the formula

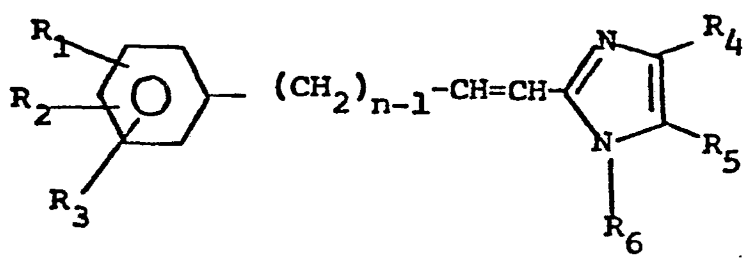

wherein $R_1$, $R_2$, $R_3$, $R_4$, $R_5$, $R_6$ and n are as defined above which is

hydrogenated to give a compound of the formula:

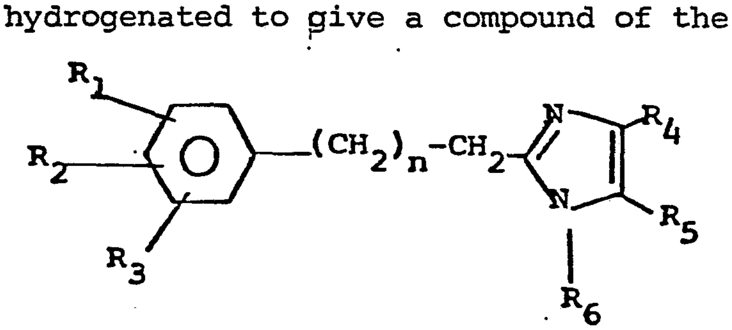

wherein $R_1$, $R_2$, $R_3$, $R_4$, $R_5$, $R_6$ and n are as defined above which, when

$R_6$ is 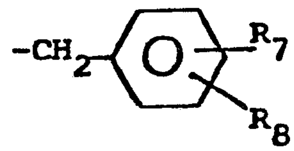 , is

reacted with sodium in liquid ammonia to give a compound of formula (I) wherein X is $-CH_2-$ and $R_6$ is hydrogen,

d) reacting an aralkylimidamide of the formula:

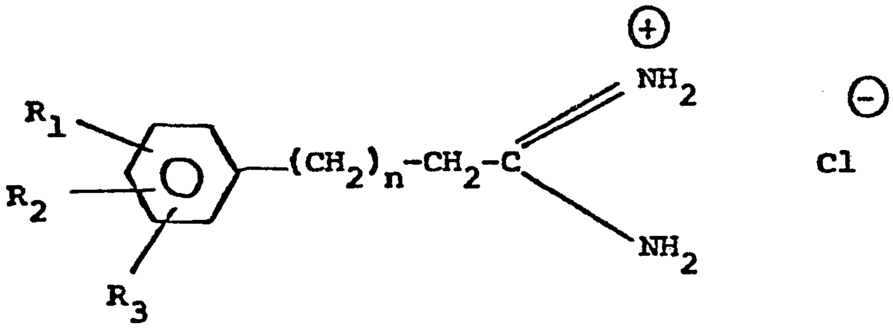

wherein $R_1$, $R_2$, $R_3$ and n are as defined above, with a compound of the formula

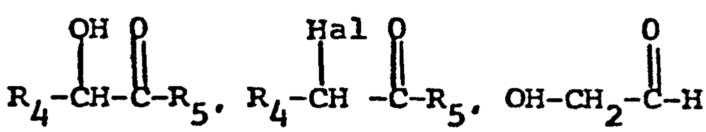 or

$$HO-CH_2-CH_2-CH(O\ Alkyl)_2$$

to form a compound of formula (I) wherein X is $-CH_2-$,

e) hydrolysing a compound of the formula:

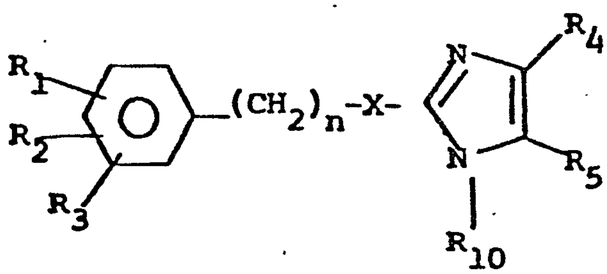

wherein $R_1$, $R_2$, $R_3$, $R_4$, $R_5$, X and n are as defined before and $R_{10}$, which also can be in the 3-position of the imidazole ring is a hydrolysable group to give a compound of the formula (I) wherein $R_6$ is hydrogen,

f) reacting an aralkylnitrile of the formula:

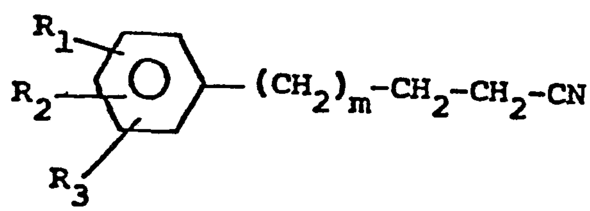

wherein $R_1$, $R_2$, $R_3$ and m are as defined above with a salt of ethylenediamine to give a compound of the formula:

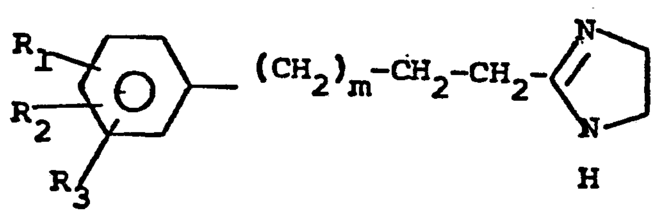

wherein $R_1$, $R_2$, $R_3$ and m are as defined above, which is aromatised to give a compound of formula (I), wherein $R_4$ and $R_5$ are hydrogen, or

g) reacting an iminoether of the formula

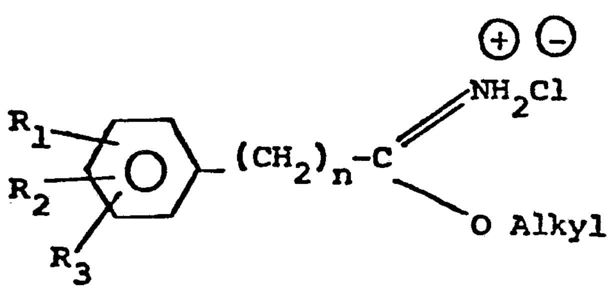

with an aminoketone, aminoacetaldehyde or its acetal of the formula:

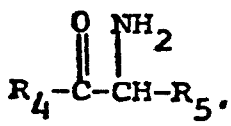, 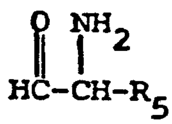 or 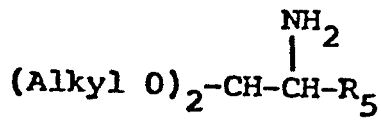

wherein $R_1$, $R_2$, $R_3$, $R_4$, $R_5$ and n are as defined above to give a compound of formula (I) wherein X is $-CH_2-$, and optionally converting a compound so obtained into an acid addition salt by conventional methods.

0064820

European Patent Office

# EUROPEAN SEARCH REPORT

Application number

EP 82 30 1971

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-1 421 774 (HOECHST)<br>* pages 1 to 3 *<br>--- | 1,3,8-10,16,30,37-39 | C 07 D 233/56<br>C 07 D 233/58<br>C 07 D 233/06<br>C 07 D 233/08<br>A 61 K 31/415 |
| X | GB-A-1 355 170 (HOFFMANN LA ROCHE)<br>* page 12 *<br>--- | 1,3 | |
| X | GB-A-1 195 454 (PFIZER)<br>* pages 1 to 4 *<br>--- | 1,4,11,12,15,37,38 | |
| Y | US-A-3 050 520 (ERNER-GREEN)<br>* columns 2 to 4 *<br>--- | 1,4,11,12,15 | TECHNICAL FIELDS SEARCHED (Int. Cl. 3) |
| Y | FR-A-2 312 244 (SCHERING)<br>* pages 18 to 22 *<br>----- | 1,4,11,12,15,37,38 | C 07 D 233/00<br>A 61 K 31/00 |

The present search report has been drawn up for all claims

| Place of search | Date of completion of the search | Examiner |
|---|---|---|
| THE HAGUE | 10-08-1982 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document